# EUROPEAN PATENT APPLICATION

(11) **EP 4 079 299 A1**
(43) Date of publication of application: **26.10.2022**
(21) Application number: 20903252.3
(22) Date of filing: 03.08.2020
(51) Int. Cl.: A61K 9/51, A61K 9/00, A61K 33/10, A61P 3/04, A61K 8/02, A61K 8/64, A61K 8/72, A61K 8/19, A61Q 19/06

(54) **LIPOLYSIS COMPOSITION USING SURFACE-MODIFIED GAS-GENERATING NANOPARTICLES**

(30) Priority: 19.12.2019 KR 20190171012
(71) Applicant: Supernova Bio Co., Ltd., Seoul 04385 (KR)
(72) Inventor: JEONG, Eun Ju, Hanam-si, Gyeonggi-do 12912 (KR); SEO, Ye Rang, Seoul 02624 (KR)
(74) Representative: Dehns
(86) International application number: PCT/KR2020/010187
(87) International publication number: WO 2021/125488

(57) **Abstract**

The present invention relates to a lipolysis composition comprising a biocompatible polymer that is surface-modified by an adipocyte-targeting or cell-penetrating peptide; and surface-modified gas-generating nanoparticles that contain fine-grained calcium carbonate crystals enclosed in the biocompatible polymer.

<110>    supernova bio Co., Ltd.

<120>    COMPOSITION FOR LIPOLYSIS USING SURFACE MODIFED GAS-GENERATING
         NANOPARTICLE

<130>    1066833

<160>    6

<170>    KoPatentIn 3.0

<210>    1
<211>    9
<212>    PRT
<213>    Artificial Sequence

<220>
<223>    P1


<400>    1
Cys Lys Gly Gly Arg Ala Lys Asp Cys
  1               5


<210>    2
<211>    13
<212>    PRT
<213>    Artificial Sequence

<220>
<223>    P2


<400>    2
Cys Lys Gly Gly Arg Ala Lys Asp Gly Gly Gly Gly Cys
  1               5                   10


<210>    3
<211>    10
<212>    PRT
<213>    Artificial Sequence

<220>
<223>    P3


<400>    3
Arg Arg Arg Arg Arg Arg Arg Arg Arg Cys
  1               5                   10


<210>    4
<211>    14
<212>    PRT
<213>    Artificial Sequence

<220>
<223>    P4


<400>    4

Arg Arg Arg Arg Arg Arg Arg Arg Arg Gly Gly Gly Gly Cys
1               5                   10

<210>    5
<211>    18
<212>    PRT
<213>    Artificial Sequence

<220>
<223>    P5

<400>    5
Cys Lys Gly Gly Arg Ala Lys Asp Arg Arg Arg Arg Arg Arg Arg Arg
1               5                   10                  15

Arg Cys

<210>    6
<211>    22
<212>    PRT
<213>    Artificial Sequence

<220>
<223>    P6

<400>    6
Cys Lys Gly Gly Arg Ala Lys Asp Arg Arg Arg Arg Arg Arg Arg Arg
1               5                   10                  15

Arg Gly Gly Gly Gly Cys
            20

## Description

### [Technical Field]

The present invention relates to a lipolysis composition using a gas-generating nanoparticle surface-modified by an optimal peptide.

### [Background Art]

Because the demand in the body contouring market is huge and continues to grow every year, various methods of liposuction and fat dissolving are being developed. Considering the shortcomings faced by liposuction performed in the past, manufacturers in the field of weight control and body contouring are shifting their development perspective away from traditional liposuction to a minimally invasive or even non-invasive liposuction method.

In the non-invasive lipolysis method, a drug or gas as a lipolytic agent is injected into an area where fat loss is hardly achieved because fat is lost very slowly through diet and exercise or cellulite (denatured fat) is formed under the skin. This method promotes fat loss in this area by dissolving the cellulite and inducing the contraction of adipose tissue and the circulation of lymphocytes, and it is mainly adopted as an injection or transdermal absorbent.

However, the conventional lipolytic agents have a problem in that they affect nerve cells, muscle cells, and bone cells that contain fat components around the adipocytes during the lipolysis process after being locally injected into the adipocytes, causing various side effects.

### [Document of Related Art]

(Patent Document 1) Korean Patent Registered Publication No. 10-1613606 (2016. 04.12)

### [DISCLOSURE]

### [Technical Problem]

The present invention is to provide a lipolysis composition including a gas-generating nanoparticle surface-modified by an adipocyte-targeting or cell-penetrating peptide for effective and safe apoptosis of adipocytes after being selectively introduced into the adipocytes.

However, the technical objects to be achieved by the present invention is not limited to the above-mentioned objects, and other objects not mentioned will be clearly understood by those skilled in the art from the following description.

### [Technical Solution]

The present invention provides a pharmaceutical composition for lipolysis, including a biocompatible polymer surface-modified by an adipocyte-targeting or cell-penetrating peptide; and a surface-modified gas-generating nanoparticle containing fine-grained calcium carbonate crystal enclosed in the biocompatible polymer.

The surface-modified gas-generating nanoparticle may be selectively introduced into adipocyte and then cause apoptosis of the adipocyte.

The surface-modified gas-generating nanoparticle may not cause apoptosis of cell other than the adipocyte.

The peptide may consist of an amino acid sequence set forth in any one of SEQ ID NOs: 1 to 6.

The surface modification may be formed by carbodiimide bonding between an amine group at an end of the biocompatible polymer and an amine group at an end of the peptide.

The biocompatible polymer may be a polymer having a structure selected from the group consisting of polylactide (PLA), polyglycolide (PGA), polylactide-polyglycolide copolymer (PLGA), starch, glycogen, chitin, peptidoglycan, lignosulfonate, tannic acid, lignin, pectin, polyethylene glycol, polyethylene oxide, polyvinyl alcohol, polyethylene oxide-polypropylene oxide block copolymer, cellulose, hemicellulose, heparin, hyaluronic acid, dextran and alginate.

A molar ratio of the peptide and the biocompatible polymer may be 0.05:1 to 1:1.

A concentration of the fine-grained calcium carbonate crystal in the pharmaceutical composition may be 0.01 (w/v) % to 10 (w/v) %.

The pharmaceutical composition may be in a form of an injection or transdermal administration.

An embodiment of the present invention provides a cosmetic composition for lipolysis, including a biocompatible polymer surface-modified by an adipocyte-targeting or cell-penetrating peptide; and a surface-modified gas-generating nanoparticle containing fine-grained calcium carbonate crystal enclosed in the biocompatible polymer.

### [Advantageous Effect]

The lipolysis composition according to the present invention includes a biocompatible polymer surface-modified by an adipocyte-targeting or cell-penetrating peptide; and a surface-modified gas-generating nanoparticle containing fine-grained calcium carbonate crystal enclosed in the biocompatible polymer. Since it is possible to effectively cause apoptosis of adipocytes by condensation gas explosion after being selectively introduced into adipocytes, it can be usefully used for lipolysis and further for preventing, improving, or treating obesity. In addition, the lipolysis composition according to the present invention does not cause apoptosis of preadipocytes, fibroblasts, muscle cells, etc. except for the adipocytes, and thus has the advantage of minimizing side effects.

### [Description of Drawings]

FIG. 1 is a photograph showing the endocytosis effect of a PLGA gas-generating nanoparticle surface-modified by optimal peptides (P1-P5, P7) into adipocytes under a fluorescence microscope.
FIG. 2 is a photograph showing the selective endocytosis effect of a PLGA gas-generating nanoparticle surface-modified by an optimal peptide (P1) into adipocytes under a confocal microscope.
FIG. 3 is a photograph showing the 3T3-L1 apoptosis effect due to a single treatment of a PLGA gas-generating nanoparticle (GNP) surface-modified by optimal peptides (P1-P5) with a scanner.
FIG. 4 is a graph showing the 3T3-L1 apoptosis effect due to multiple treatments of a PLGA gas-generating nanoparticle (GNP) surface-modified with optimal peptides (P1, P3, P7).
FIG. 5 is a graph showing the 3T3-L1 apoptosis effect of a PLGA gas-generating nanoparticle (GNP) surface-modified by an optimal peptide (P1) of various molar ratios.
FIG. 6 is a graph showing the 3T3-L1 apoptosis effect according to various concentrations of fine-grained calcium carbonate crystal.
FIG. 7 is a graph showing the selective 3T3-L1 apoptosis effect of a PLGA gas-generating nanoparticle (GNP) surface-modified by an optimal peptide (P1).
FIG. 8 is a graph showing the fat reduction effect of a PLGA gas-generating nanoparticle (GNP) surface-modified by an optimal peptide (P1) in diet-induced obese mice.

### [Mode for Invention]

The present inventors have prepared a surface-modified gas-generating nanoparticle, in which an adipocyte-targeting or cell-penetrating peptide as an optimal peptide is introduced on the surface of gas-generating nanoparticle, and the inventors has confirmed that the nanoparticle can effectively cause apoptosis of adipocytes after selectively transfected into the adipocytes, thereby completed the present invention.

The present invention provides a pharmaceutical composition for lipolysis including a biocompatible polymer surface-modified by an adipocyte-targeting or cell-penetrating peptide; and a surface-modified gas-generating nanoparticle containing fine-grained calcium carbonate crystal enclosed in the biocompatible polymer.

Alternatively, the present invention provides a pharmaceutical composition for preventing or treating obesity including a biocompatible polymer surface-modified by an adipocyte-targeting or cell-penetrating peptide; and a surface-modified gas-generating nanoparticle containing fine-grained calcium carbonate crystal enclosed in the biocompatible polymer.

First, the gas-generating nanoparticle used in the present invention includes a biocompatible polymer; and fine-grained calcium carbonate crystal enclosed in the biocompatible polymer.

The term "fine-grained calcium carbonate crystal" used herein is to be distinguished from precipitated calcium carbonate prepared by chemical processing, and generally refers to manufactured by mechanically grinding and classifying high-purity crystalline limestone.

In addition, the term "biocompatible polymer" used herein refers to a polymer having tissue compatibility and blood compatibility that does not destroy tissue or coagulate blood in contact with living tissue or blood, and refers to a polymer capable of forming a spherical structure of hard shell in which fine-grained calcium carbonate crystal is enclosed by an emulsion method. Specifically, the biocompatible polymer may be a polymer having a structure selected from the group consisting of polylactide (PLA), polyglycolide (PGA), polylactide-polyglycolide copolymer (PLGA), starch, glycogen, chitin, peptidoglycan, lignosulfonate, tannic acid, lignin, pectin, polyethylene glycol, polyethylene oxide, polyvinyl alcohol, polyethylene oxide-polypropylene oxide block copolymer, cellulose, hemicellulose, heparin, hyaluronic acid, dextran and alginate.

The gas-generating nanoparticle has a form in which fine-grained calcium carbonate crystal is embedded without flow in a spherical structure of a hard shell of a biocompatible polymer, which refers to a structure in which the fine-grained calcium carbonate crystal is evenly embedded without any significant difference in distribution probability from the center to outer surface of the spherical structure.

In the gas-generating nanoparticle, the weight ratio of the fine-grained calcium carbonate crystal and the biocompatible polymer may be 0.001:1 to 1:1, preferably 0.005:1 to 0.5:1, and more preferably 0.005:1 to 0.05: 1, but is not limited thereto. In the gas-generating nanoparticle, when the content of the fine-grained calcium carbonate crystal is too low, there is a problem in that the generation of carbon dioxide gas is not sufficient, and when the content of the biocompatible polymer is too low, there is a problem in that the spherical structure of a hard-film is not robust.

The gas-generating nanoparticle causes a reaction in which the fine-grained calcium carbonate enclosed in the biocompatible polymer generates carbon dioxide gas in an acidic environment. That is, when the fine-grained calcium carbonate crystal generates carbon dioxide gas, the internal pressure of the gas-generating nanoparticle gradually increases, which ultimately induces the explosion of the gas-generating nanoparticle. Due to this condensed gas explosion, cell apoptosis can be effectively caused. However, without introduction of an optimal peptide to the surface of the gas-generating nanoparticle, side effects due to indiscriminate cell apoptosis are problematic.

The gas-generating nanoparticle can generate a synergistic effect with calcium carbonate crystal by additionally enclosing a known lipolytic agent or an anti-obesity agent inside the biocompatible polymer.

Accordingly, a pharmaceutical composition for lipolysis or for preventing or treating obesity according to the present invention includes a surface-modified gas-generating nanoparticle. In the surface-modified gas-generating nanoparticle, an adipocyte-targeting or cell-penetrating peptide is introduced as an optimal peptide on the surface of the gas-generating nanoparticle.

Accordingly, the surface-modified gas-generating nanoparticle can be selectively introduced into adipocytes and then causes apoptosis of the adipocytes. Meanwhile, the surface-modified gas-generating nanoparticle may not cause apoptosis or minimize the apoptosis of cells other than adipocytes (preadipocytes, fibroblasts, muscle cells, etc.). Therefore, it can be usefully used for lipolysis, and further for preventing, improving or treating obesity.

The surface modification may be accomplished by carbodiimide bonding between an amine group at the end of the biocompatible polymer and an amine group at the end of the peptide. In this case, the terminal amine group of the biocompatible polymer may be introduced through activation of a carboxyl group present in the biocompatible polymer, and may react with the terminal amine group of the peptide (cysteine) to form the carbodiimide bond.

As the optimal peptide, an adipocyte-targeting or cell-penetrating peptide is used, and it may consist of the amino acid sequence set forth in any one of SEQ ID NOs: 1 to 6.

Specifically, as the optimal peptide, an adipocyte-targeting peptide consisting of the amino acid sequence set forth in SEQ ID NO: 1 or 2 alone; a cell-penetrating peptide consisting of the amino acid sequence set forth in SEQ ID NO: 3 or 4 alone; a linearity of the adipocyte-targeting peptide and the cell-penetrating peptide consisting of the amino acid sequence set forth in SEQ ID NO: 5 or 6; or a combination of the adipocyte-targeting peptide and the cell-penetrating peptide consisting of the amino acid sequence set forth in SEQ ID NO: 7 (wherein a molar ratio = 1:100 to 100:1, preferred molar ratio = 1:10 to 10:1) may be used. In particular, the optimal peptide may be preferably consisted of the amino acid sequence set forth in SEQ ID NO: 1 in terms of maximizing the apoptosis effect of adipocytes, but is not limited thereto.

The molar ratio of the peptide and the biocompatible polymer may be 0.05:1 to 1:1, preferably 0.5:1 to 1:1, but is not limited thereto. As the content of the peptide increases, there is an advantage in that the apoptosis effect of adipocytes can be increased. However, since the peptide can be introduced through the end functional group of the biocompatible polymer, the maximum molar ratio of the peptide and the biocompatible polymer may be 1:1. Moreover, when the peptide consists of the amino acid sequence set forth in SEQ ID NO: 3 or 4, as the content of the peptide increases, the surface-modified gas-generating nanoparticles generated agglomerate, thereby greatly reducing the stability.

The surface-modified gas-generating nanoparticle may have a diameter of 100 nm to 250 nm, preferably 120 nm to 250 nm, more preferably 150 nm to 250 nm, and most preferably 160 nm to 230 nm, but is not limited thereto. It is possible to effectively cause the apoptosis of adipocytes within this diameter range.

The concentration of the fine-grained calcium carbonate crystal in the pharmaceutical composition may be 0.01 (w/v) % to 10 (w/v) %, preferably 0.05 (w/v) % to 10 (w/v) %, but is not limited thereto. As such, as the concentration of the fine-grained calcium carbonate crystal in the pharmaceutical composition increases, the apoptosis effect of adipocytes may be increased.

The pharmaceutical composition may be formulated in oral or parenteral form according to a commonly used method, respectively, and it is preferably formulated in the form of an injection injected into a local site (e.g., a fat pad) or a transdermal administration form injected using a microneedle or patch, but is not limited thereto. When the pharmaceutical composition is formulated in the form of an injection or oral administration, there is an advantage in that it can replace a conventional carboxytherapy procedure, mesotherapy, phosphatidylcholine injection, deoxycholine injection, hypotonic lipo-dissolution injection, etc.

For such formulation, an appropriate carrier, excipient or diluent commonly used in the preparation of the pharmaceutical composition may be included.

As the carrier or excipient or diluent, there are various compounds or mixtures including lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil and the like.

In particular, parenteral formulation (especially, injection type) includes sterile aqueous solution, non-aqueous formulation, suspension, emulsion, lyophilizing agent and suppository. As the non-aqueous solvent and the suspension, propylene glycol, polyethylene glycol, vegetable oil such as olive oil, and injectable ester such as ethyl oleate can be used. As the base of the suppository, witepsol, macrogol, tween 61, cacao butter, laurinum, glycerolgelatin and the like can be used.

The preferred dosage of the pharmaceutical composition varies depending on the patient's condition, body weight, degree of disease, drug form, administration route and period, but may be appropriately selected by those skilled in the art. However, for a desirable effect, it may be administered at 0.0001 to 2,000 mg/kg per day, preferably 0.001 to 2,000 mg/kg. The composition may be administered once a day, or may be administered in divided doses several times a day. However, the scope of the present invention is not limited by the dosage.

The pharmaceutical composition may be administered to mammals such as rats, mice, livestock, and humans by various routes. The administration may be performed in any mode, for example, oral, dermal, fat pad, rectal or intravenous, intramuscular, subcutaneous, intrauterine dural or intracerebroventricular injection.

In addition, the present invention provides a cosmetic composition for lipolysis including a biocompatible polymer surface-modified by an adipocyte-targeting or cell-penetrating peptide; and a surface-modified gas-generating nanoparticle containing fine-grained calcium carbonate crystal enclosed in the biocompatible polymer.

Alternatively, the present invention provides a cosmetic composition for preventing or improving obesity including a biocompatible polymer surface-modified by an adipocyte-targeting or cell-penetrating peptide; and a surface-modified gas-generating nanoparticle containing fine-grained calcium carbonate crystal enclosed in the biocompatible polymer.

The cosmetic composition according to the present invention includes, but is not limited to, a composition selected from the group consisting of a water-soluble vitamin, an oil-soluble vitamin, a high molecular peptide, a high molecular polysaccharide, a sphingolipid, and a seaweed extract.

The water-soluble vitamin may be used without limitation as long as it can be blended with cosmetics. Preferably, the water-soluble vitamin may include vitamin B1, vitamin B2, vitamin B6, pyridoxine, pyridoxine hydrochloride, vitamin B 12, pantothenic acid, nicotinic acid, nicotinic acid amide, folic acid, vitamin C, vitamin H, etc., their salts (thiamine hydrochloride, sodium ascorbate salt, etc.) and derivatives (ascorbic acid-2-phosphate sodium, ascorbic acid-2-phosphate magnesium, etc.), but is not limited thereto.

The oil-soluble vitamin may be used without limitation as long as it can be blended with cosmetics. Preferably, the oil-soluble vitamin may include vitamin A, carotin, vitamin D2, vitamin D3, vitamin E (d1-α tocopherol, d-α tocopherol) and derivatives thereof (ascorbic acid palmitate, ascorbic acid stearate, ascorbic acid dipalmitate, acetic acid d1-α tocopherol, nitotinic acid d1-α tocopherol vitamin E, DL-pantothenyl alcohol, D-pantothenyl alcohol, pantothenyl ethyl ether, etc.), but is not limited thereto.

The high molecular peptide may be used without limitation as long as it can be blended with cosmetics. Preferably, the high molecular peptide may include collagen, hydrolyzed collagen, gelatin, elastin, hydrolyzed elastin or keratin, but is not limited thereto.

The high molecular polysaccharide may be used without limitation as long as it can be blended with cosmetics. Preferably, the high molecular polysaccharide may include hydroxyethyl cellulose, xanthan gum, sodium hyaluronate, chondroitin sulfate or a salt thereof (sodium salt, etc.), but is not limited thereto.

The sphingolipid may be used without limitation as long as it can be blended with cosmetics. Preferably, the sphingolipid may include ceramide, phytosphingosine, and glycosphingolipid, but is not limited thereto.

The seaweed extract may be used without limitation as long as it can be blended with cosmetics. Brown algae extract, red algae extract or green algae extract is preferable, and carrageenan, arginic acid, sodium alginate or potassium arginate purified from these seaweed extracts is preferable, However, the seaweed extract is not limited thereto.

In addition to the essential ingredients, the cosmetic composition may contain other ingredients that are usually blended with cosmetics, if necessary. Examples of the other ingredients include a fat component, a humectant, an emollient, a surfactant, organic and inorganic pigments, an organic powder, a UV absorber, an antiseptic, a bactericide, an antioxidant, a plant extract, a pH adjusting agent, an alcohol, a colorant, an aromatic, a blood flow stimulant, a cooling agent, an antiperspirant, or purified water are preferred, but are not limited thereto.

The fat component may preferably include ester-based fat, hydrocarbon-based fat, silicone-based fat, fluorine-based fat, animal fat, or vegetable fat, but is not limited thereto. Preferably, the ester-based fat may include tri 2-ethylhexaneglyceryl, 2-ethylhexanecetyl, myristic acid isopropyl, myristic acid butyl, palmitic acid isopropyl, stearic acid ethyl, palmitic acid octyl, isostearic acid isocetyl, stearic acid butyl, linoleic acid ethyl, linoleic acid isopropyl, oleic acid ethyl, myristic acid isocetyl, myristic acid isostearyl, palmitic acid isostearyl, myristic acid octyldodecyl, isostearic acid isocetyl, sebacic acid diethyl, adipic acid diisopropyl, neopentanoic acid isoalkyl, tri(caprylic, capric acid)glyceryl, tri2-ethylhexanetrimethylolpropane, triisostearic acid trimethylolpropane, tetra2-ethylhexanepentaerythritol, caprylic acid cetyl, lauric acid decyl, lauric acid hexyl, myristic acid decyl, myristic acid myristyl, myristic acid cetyl, stearic acid stearyl, oleic acid decyl, ricinoleic acid cetyl, lauric acid isostearyl, myristic acid isotridecyl, palmitic acid isocetyl, stearic acid octyl, stearic acid isocetyl, oleic acid isodecyl, oleic acid octyldodecyl, linoleic acid octyldodecyl, isostearic acid isopropyl, 2-ethylhexanecetostearyl, 2-ethylhexanestearyl, isostearic acid hexyl, dioctanoic acid ethyleneglycol, dioleic acid ethyleneglycol, dicapric acid propylene glycol, di(caprylic, capric acid)propylene glycol, dicapric acid propylene glycol, dicapric acid neopentylglycol, dioctanoic acid neopentylglycol, tricaprylic acid glyceryl, triundecylic acid glyceryl, triisopalmitic acid glyceryl, triisostearic acid glyceryl, neopentanoic acid octyldodecyl, octanoic acid isostearyl, isononanoic acid octyl, neodecanoic acid hexyldecyl, neodecanoic acid octyldodecyl, isostearic acid isocetyl, isostearic acid isostearyl, isostearic acid octyldecyl, polyglycerineoleic acid ester, polyglycerineisostearic acid ester, citric acid triisocetyl, citric acid triisoalkyl, citric acid triisooctyl, lactic acid lauryl, lactic acid myristyl, lactic acid cetyl, lactic acid octyldecyl, citric acid triethyl, citric acid acetyltriethyl, citric acid acetyltributyl, citric acid trioctyl, malic acid diiostearyl, hydroxystearic acid 2-ethylhexyl, succinic acid di-2-ethylhexyl, adipic acid diisobutyl, sebacic acid diisopropyl, sebacic acid dioctyl, stearic acid cholesteryl, isostearic acid cholesteryl, hydroxystearic acid cholesteryl, oleic acid cholesteryl, oleic acid dihydrocholesteryl, isostearic acid phytosteryl, oleic acid phytosteryl, 12-stearoylhydroxystearic acid isocetyl, 12-stearoylhydroxystearic acid stearyl, 12-stearoylhydroxystearic acid isostearyl, and the like, but is not limited thereto.

The hydrocarbon-based fat may preferably include squalene, liquid paraffin, α-olefin oligomer, isoparaffin, ceresin, paraffin, liquid isoparaffin, polybudene, microcrystalline wax or vaselin, but is not limited thereto.

The silicone-based fat may preferably include polymethylsilicone, methylphenylsilicone, methylcyclopolysiloxane, octamethylpolysiloxane, decamethylpolysiloxane, dodecamethylcyclosiloxane, dimethylsiloxane methylcetyloxysiloxane copolymer, dimethylsiloxane methylstearoxysiloxane copolymer, alkyl-modified silicone oil or amino-modified silicone oil, but is not limited thereto.

The fluorine-based fat may preferably include perfluoropolyether, but is not limited thereto.

The animal or vegetable fat may preferably include avocado oil, almond oil, olive oil, sesame oil, rice bran oil, safflower oil, soybean oil, corn oil, rapeseed oil, apricot oil, palm kernel oil, palm oil, castor oil, sunflower oil, grapeseed oil, cottonseed oil, coconut oil, kukui nut oil, wheat germ oil, rice germ oil, shea butter, evening-primrose oil, macadamia nut oil, meadowfoam oil, egg yolk oil, tallow, horse oil, mink oil, orange roughy oil, jojoba oil, candelilla wax, carnauba wax, liquid lanolin, hydrogenated castor oil etc., but is not limited thereto.

The humectant may preferably include a water-soluble low molecular humectant, a fat-soluble molecular humectant, a water-soluble polymer, or a fat-soluble polymer, etc., but is not limited thereto. The water-soluble low molecular humectant may preferably include serine, glutamine, sorbitol, mannitol, pyrrolidone-sodium carboxylate, glycerin, propylene glycol, 1,3-butyleneglycol, ethylene glycol, polyethylene glycol B (degree of polymerization, n=2 or more), polypropylene glycol (degree of polymerization, n=2 or more), polyglycerin B (degree of polymerization, n=2 or more), lactic acid or lactate, but is not limited thereto. The fat-soluble low molecular humectant may preferably include cholesterol or cholesterol ester, but is not limited thereto. The water-soluble polymer may preferably include carboxyvinyl polymer, polyaspartate, tragacanth, xanthan gum, methyl cellulose, hydroxymethyl cellulose, hydroxyethyl cellulose, hydroxypropyl cellulose, carboxymethyl cellulose, water-soluble chitin, chitonic acid, or dextrin, but is not limited thereto. The fat-soluble polymer may preferably include polyvinylpyrrolidone eicosene copolymer, polyvinylpyrrolidone hexadecene copolymer, nitrocellulose, dextrin fatty acid ester or polymer silicone, but is not limited thereto. The emollient may preferably include long-chain acyl glutamic acid cholesteryl ester, hydroxystearic acid cholesteryl, 12-hydroxystearic acid, stearic acid, rosin acid, or lanolin fatty acid cholesteryl ester, but is not limited thereto.

The surfactant may preferably include a nonionic surfactant, an anionic surfactant, a cationic surfactant, or an amphoteric surfactant, but is not limited thereto. The nonionic surfactant may preferably include self-emulsified monostearic acid glycerine, propylene glycol fatty acid ester, glycerin fatty acid ester, polyglycerine fatty acid ester, sorbitan fatty acid ester, polyoxyethylene (POE) sorbitan fatty acid ester, POE sorbite fatty acid ester, POE glycerine fatty acid ester, POE alkylether, POE fatty acid ester, POE hydrogenated castor oil, POE castor oil, polyoxyethylenepolyoxypropylene(POEPOP) copolymer, POEPOP alkylether, polyether-modified silicone, lauric acid alkanol amide, alkylamine oxide or hydrogenated soybean phospholipid, but is not limited thereto. The anionic surfactant may preferably include fatty acid soap, α-acylsulfonate, alkylsulfonate, alkylallylsulfonate, alkylnaphthalenesulfonate, alkylsulfate, POE alkylethersulfate, alkylamidesulfate, alkylphosphate, POE alkylphosphate, alkylamidephosphate, alkyloylalkyltaurate, N-acylamino acid salt, POE alkylethercarboxylate, alkylsulfosuccinate, sodium alkylsulfoacetate, acylated hydrolyzed collagen peptide salt or perfluoroalkyl phosphate ester, but is not limited thereto. The cationic surfactant may preferably include alkyltrimethyl ammonium chloride, stearyltrimethyl ammonium chloride, stearyltrimethyl ammonium bromide, cetostearyltrimethyl ammonium chloride, distearyldimethyl ammonium chloride, stearyldimethylbenzyl ammonium chloride, behenyltrimethyl ammonium bromide, benzalkonium chloride, diethylaminoethylamide stearate, dimethylaminopropylamide stearate or quaternary ammonium salt derivative of lanolin, but not limited thereto. The amphoteric surfactant may preferably include carboxybetaine-type, amidebetaine-type, sulfobetaine-type, hydroxy sulfobetaine-type, amidesulfobetaine-type, phosphobetaine-type, aminocarboxylate-type, imidazoline derivative-type, amideamine-type amphoteric surfactant, but not limited thereto.

The organic and inorganic pigments may preferably include an inorganic pigment such as silicic acid, silicic anhydride, magnesium silicate, talc, sericite, mica, kaolin, bengala, clay, bentonite, titan-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and complexes thereof; an organic pigment such as polyamide, polyester, polypropylene, polystyrene, polyurethane, vinyl resin, urea resin, phenol resin, fluorine resin, silica resin, acrylic resin, melamine resin, epoxy resin, polycarbonate resin, divinylbenzene styrene copolymer, silk powder, cellulose, CI pigment yellow, and CI pigment orange, and a complex pigment of the inorganic pigment and the organic pigment, but are not limited thereto.

The organic powder may preferably include a metallic soap such as calcium stearate; alkyl phosphate metal salt such as zinc sodium cetylate, zinc laurylate, and calcium laurylate; polyvalent acylamino acid metal salt such as N-lauroyl-β-alanine calcium, N-lauroyl-β-alanine zinc, and N-lauroyl glycine calcium; polyvalent amide sulfonic acid metal salt such as N-lauroyl-taurine calcium and N-palmitoyl-taurine calcium; N-acyl basic amino acid such as N-ε-lauroyl-L-lysine, N-ε-palmitoyllysine, N-α-palmitoyl ornithine, N-α-lauroylarginine, and N-α-hydrogenated tallow fatty acid acyl arginine; N-acylpolypeptide such as N-lauroylglycylglycine; α-amino fatty acid such as α-amino caprylic acid and α-amino lauric acid; or polyethylene, polypropylene, nylon, polymethylmethacrylate, polystyrene, divinylbenzene styrene copolymer, tetrafluoroethylene, but is not limited thereto.

The UV absorber may preferably include para-aminobenzoic acid, ethyl para-aminobenzoate, amyl para-aminobenzoate, octyl para-aminobenzoate, salicylic acid ethylene glycol, phenyl salicylate, octyl salicylate, benzyl salicylate, butylphenyl salicylate, homomentyl salicylate, benzyl cinnamate, 2-ethoxyethyl para-methoxy cinnamate, octyl para-methoxy cinnamate, mono-2-ethyl hexane glyceryl di-para-methoxy cinnamate, isopropyl para-methoxy cinnamate, diisopropyl diisopropyl cinnamic acid ester mixture, urocanic acid, ethyl urocanate, hydroxy methoxybenzophenone, hydroxy methoxybenzophenone sulfonic acid and salts thereof, dihydroxy methoxybenzophenone, sodium dihydroxy methoxybenzophenone disulfonate, dihydroxy benzophenone, tetrahydroxy benzophenone, 4-tert-butyl-4'-methoxy dibenzoylmethane, 2,4,6-trianilino-p-(carbo-2'-ethylhexyl-1'-oxy)-1,3,5-triazine or 2-(2-hydroxy-5-methylphenyl)benzotriazole, but is not limited thereto.

The bactericide may preferably include hinokitiol, triclosan, trichlorohydroxydiphenyl ether, chlorhexidine gluconate, phenoxyethanol, resorcin, isopropylmethylphenol, azulene, salicylic acid, zincpyrithione, benzalkonium chloride, photosensitizer 301, sodium mononitroguaiacol or undecylenic acid, but is not limited thereto.

The antioxidant may preferably include butylhydroxy anisole, propyl gallate, or erythorbic acid, but is not limited thereto.

The pH adjusting agent may preferably include citric acid, sodium citrate, malic acid, sodium malate, fumaric acid, sodium fumarate, succinic acid, sodium succinate, sodium hydroxide or disodium hydrogen phosphate, but is not limited thereto.

The alcohol may preferably include a higher alcohol such as cetyl alcohol, but is not limited thereto.

In addition, a blending component which may be added herein is not limited to the above-described components, and any component may be blended in such a range that the objects and effects of the present invention are not hindered. For example, the component may be preferably blended in an amount of 0.01 to 5 % by weight, and more preferably an amount of 0.01 to 3 % by weight, based on the total weight of the composition, but is not limited thereto.

The cosmetic composition may be prepared in the form of solution, emulsion, or viscous mixture, but is not limited thereto.

The components included in the cosmetic composition may include components generally used in cosmetic compositions in addition to the above compounds as an active ingredient, and may include commonly used adjuvants and carriers such as a stabilizing agent, a solubilizing agent, a vitamin, a pigment and an aromatic, but is not limited thereto.

The cosmetic composition may be prepared in any formulation which is generally prepared in the art, and may be prepared in a milky lotion, a cream, a cosmetic lotion, a pack, a foundation, a lotion, a cosmetic liquid or a hair cosmetic, but is not limited thereto. Specifically, the cosmetic composition may include formulations of a skin lotion, a skin softener, a skin toner, an astringent, a lotion, a milk lotion, a moisture lotion, a nourishing lotion, a massage cream, a nourishing cream, a moisture cream, a hand cream, a foundation, an essence, a nourishing essence, a pack, a soap, a cleansing foam, a cleansing lotion, a cleansing cream, a body lotion, or a body cleanser, but is not limited thereto.

When the formulation is in the form of a paste, cream or gel, the carrier component may include an animal fiber, a vegetable fiber, wax, paraffin, starch, tragant, a cellulose derivative, polyethylene glycol, silicone, bentonite, silica, talc or zinc oxide, but is not limited thereto.

When the formulation is in the form of powder or spray, lactose, talc, silica, aluminum hydroxide, calcium silicate or polyamide powder may be used as the carrier component. In particular, when the formulation is in the form of spray, the formulation may further include a propellent such as chlorofluorohydrocarbon, propane/butane or dimethyl ether, but is not limited thereto.

When the formulation is in the form of solution or emulsion, a solvent, a solvating agent or an emulsifying agent may be used as the carrier component. For example, the carrier component may include water, ethanol, isopropanol, ethyl carbonate, ethyl acetate, benzyl alcohol, benzyl benzoate, propylene glycol, 1,3- butylglycol oil, aliphatic glycerol ester, polyethylene glycol, or fatty acid ester of sorbitan, but is not limited thereto.

When the formulation is in the form of suspension, the carrier component may include a liquid diluent such as water, ethanol or propylene glycol, a suspension such as ethoxylated isostearyl alcohol, polyoxyethylene sorbitol ester and polyoxyethylene sorbitan ester, microcrystalline cellulose, aluminum metahydroxide, bentonite, agar or tragant, but is not limited thereto.

When the formulation is in the form of surfactant-containing cleansing, the carrier component may include aliphatic alcohol sulfate, aliphatic alcohol ether sulfate, sulfosuccinic acid monoester, isethionate, imidazolium derivative, methyltaurate, sarcosinate, fatty acid amide ether sulfate, alkylamido betaine, aliphatic alcohol, fatty acid glyceride, fatty acid diethanolamide, vegetable oil, linolin derivative, or ethoxylated glycerol fatty acid ester, but is not limited thereto.

As described above, the lipolysis composition according to the present invention includes a biocompatible polymer surface-modified by an adipocyte-targeting or cell-penetrating peptide; and a surface-modified gas-generating nanoparticle containing fine-grained calcium carbonate crystal enclosed in the biocompatible polymer. After being selectively introduced into adipocytes, the composition can effectively cause the apoptosis of adipocytes, so that it can be usefully used for lipolysis, and further for preventing, improving or treating obesity. In addition, the lipolysis composition according to the present invention does not cause the apoptosis of preadipocytes, fibroblasts, muscle cells, etc. except for the adipocytes, and thus has the advantage of minimizing side effects.

Furthermore, the present invention relates to the use of the biocompatible polymer surface-modified by an adipocyte-targeting or cell-penetrating peptide; and the surface-modified gas-generating nanoparticle containing fine-grained calcium carbonate crystal enclosed in the biocompatible polymer in a pharmaceutical composition for lipolysis.

In addition, the present invention provides the use of the biocompatible polymer surface-modified by adipocyte-targeting or cell-penetrating peptide; and the surface-modified gas-generating nanoparticle containing fine-grained calcium carbonate crystal enclosed in the biocompatible polymer in a cosmetic composition for lipolysis.

In addition, the present invention provides a method for decomposing fat including the steps of administering to a subject the biocompatible polymer surface-modified by an adipocyte-targeting or cell-penetrating peptide; and the surface-modified gas-generating nanoparticle containing fine-grained calcium carbonate crystal enclosed in the biocompatible polymer.

The term "subject" used herein refers to a subject in need of treatment, and more specifically, a mammal such as a human, or a non-human primate, a mouse, a rat, a dog, a cat, a horse, a cow, and the like.

Hereinafter, preferred examples are presented to help the understanding of the present invention. However, the following examples are only provided for easier understanding of the present invention, and the contents of the present invention are not limited by the following examples.

### [Examples]

### Example 1: Preparation of poly(lactide-co-glycolide) (PLGA) gas-generating nanoparticle (GNP) surface-modified by optimal peptide (P1-P7)

According to the method disclosed in Example 2 of Korean Patent Registered Publication No. 10-1613606, the PLGA gas-generating nanoparticle was prepared. The prepared PLGA gas-generating nanoparticle and the optimal peptide (P1-P7) were subjected to a carbodiimide binding reaction to modify the surface of the nanoparticle.

Specifically, the PLGA gas-generating nanoparticle (0.5 mM) was suspended in 0.1 M MES buffer (pH 7.0), and EDC (0.5 mM)/sulfo-NHS (0.5 mM) was added to activate the terminal carboxyl group present in the PLGA gas-generating nanoparticle. The activated PLGA gas-generating nanoparticle was reacted with ethylenediamine (0.5 mM) for one day, and then, washed three times with deionized water, and then resuspended in MES buffer (pH 7.0). EDC/sulfo-NHS and the optimal peptide (molar ratio of optimal peptide: PLGA = 1:1) described in Table 1 below were added to a solution in which the PLGA gas-generating nanoparticle functionalized with primary amine groups was suspended and reacted, and washed with deionized water, and then freeze-dried.

**[Table 1]**

| No. | Amino Acid Sequence | SEQ ID NO |
|---|---|---|
| P1 | CKGGRAKDC | SEQ ID NO: 1 |
| P2 | CKGGRAKDGGGGC | SEQ ID NO: 2 |
| P3 | rrrrrrrrrC | SEQ ID NO: 3 |
| P4 | rrrrrrrrrGGGGC | SEQ ID NO: 4 |
| P5 | CKGGRAKDrrrrrrrrrC(linear of P1 and P3) | SEQ ID NO: 5 |
| P6 | CKGGRAKDrrrrrrrrrGGGGC(linear of P1 and P4) | SEQ ID NO: 6 |
| P7 | CKGGRAKDC, rrrrrrrrrC(combination of P1 and P3; molar ratio = 1:10 to 10:1) | SEQ ID NOs: 1, 3 |

### Example 2: 3T3-L1 cell culture and differentiation

3T3-L1 undifferentiated cells were cultured in a confocal dish or 96-well plate, and cultured in DMEM (Dulbecco's modified Eagle's medium) containing 10 % bovine calf serum (BCS) at 37 °C under 5 % CO₂. After reached confluence, the 3T3-L1 cells were grown until the second day (Day 0), and treated with a growth medium containing 10 % fetal bovine serum (FBS) with a medium containing MDI (0.5 µM methylisobutylzanrin, 1 µM dexamethasone and 10 mg/L insulin) for 2 days. Then, after treatment for an additional 2 days in a growth medium containing 10 mg/L insulin alone, the growth medium was exchanged every 2 days and cultured, and all experiments were started on the seventh day after differentiation induction.

### Example 3: Endocytosis analysis of PLGA nanoparticle (PNP) surface-modified by optimal peptides (P1 to P5, P7) in 3T3-L1 cells

In order to analyze the endocytosis of the PLGA nanoparticle surface-modified with an optimal peptide in the 3T3-L1 cells, a fluorescent substance was introduced on the surface of the PLGA nanoparticle surface modified with the optimal peptides [P1 to P5, P7 (wherein a molar ratio of a combination of P1 and P3 is 1:1)].

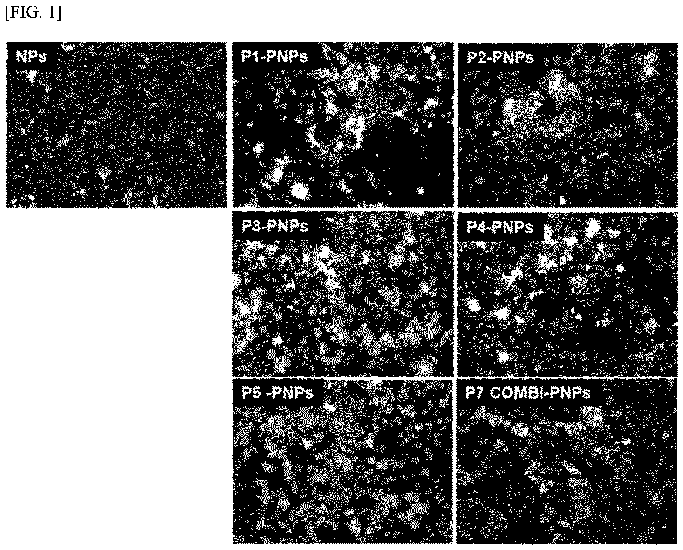

Specifically, the 3T3-L1 cells were treated with the PLGA nanoparticle surface-modified by the optimal peptides [P1 to P5, P7 (wherein a molar ration of a combination of P1 and P3 is 1:1)], in which the fluorescent substance (Cy5) was introduced, at the concentration of 0.5 mg/ml for 6 hours. Then, LysoTracker Green DND-26 for staining lysosomes was added to confirm the endocytosis into the 3T3-L1 cells. The medium was removed, and the cells were washed three times with PBS, fixed with 4 % formaldehyde, stained with DAPI for nucleus staining, mounted, and observed with a fluorescence microscope (TE2000-E) (Nikon) (FIG. 1).

As shown in FIG. 1, it was confirmed that the PLGA nanoparticle surface-modified with the adipocyte-targeting or cell-penetrating peptides [P1 to P5, P7 (wherein a molar ration of a combination of P1 and P3 is 1:1)] exhibited the improved endocytosis effects in the 3T3-L1 cells, compared with the PLGA nanoparticle with non-modified surface. This means that the introduction of the adipocyte-targeting or cell-penetrating peptide into the PLGA nanoparticle improved intracellular delivery ability.

On the other hand, the PLGA nanoparticle surface-modified by introducing an adipocyte-targeting peptide and a cell-penetrating peptide in a combined form [P7 (wherein a molar ratio of a combination of P1 and P3 is 1:1)] exhibited a higher colocalization pattern, which confirmed the improvement of the endocytosis effect into the 3T3-L1 cells, compared to the PLGA nanoparticle surface-modified by introducing the adipocyte-targeting peptide and the cell-penetrating peptide linearly in linear (P5).

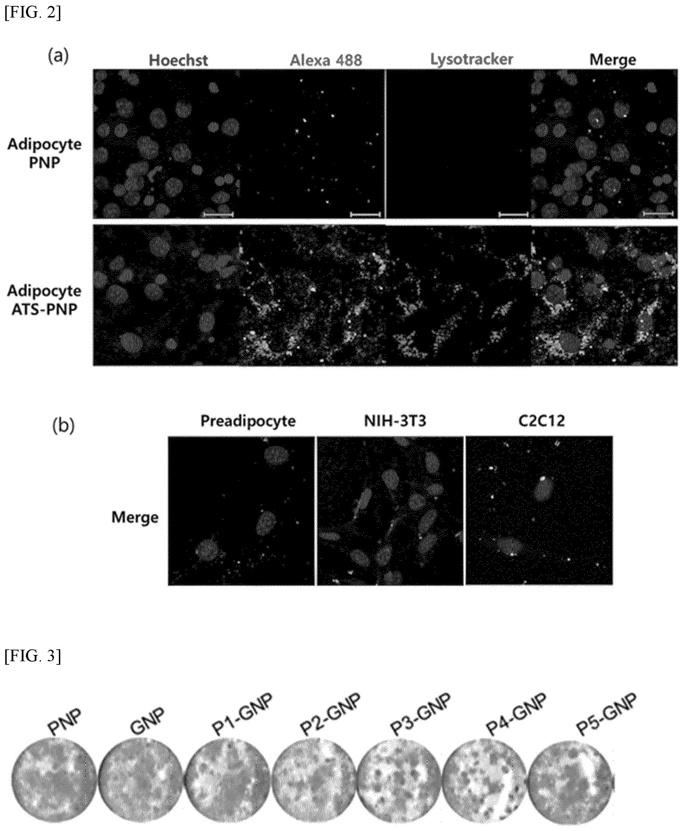

On the other hand, the 3T3-L1 cells, 3T3-L1 progenitor cells, NIH3T3 fibroblasts, and C2C12 myocytes were introduced with a fluorescent substance (Hoechst, Alexa 488, Lysotracker), and were treated with the surface-modified PLGA nanoparticle with the optimal peptide (P1) at a concentration of 0.5 mg/ml for 24 hours, and LysoTracker Red DND-99 for staining lysosomes was added to confirm endocytosis. After removing the medium and washing 3 times with PBS, the cells were fixed with 4 % formaldehyde, the nucleus of the cells were stained using Hoechst, the cells were mounted and observed with a confocal microscope (TCS SP5) (Leica) (FIG. 2).

As shown in FIG. 2 , in the case of the PLGA nanoparticle surface-modified with the adipocyte-targeting peptide (P1), it was confirmed that the delivery ability into the 3T3-L1 cells was high, but it was hardly delivered into the 3T3-L1 progenitor cells, the NIH3T3 fibroblasts, the C2C12 muscle. This can be considered to be selectively delivered only to the 3T3-L1 cells due to the adipocyte-targeting peptide (P1) that specifically binds to prohibitin, which is highly expressed on the adipocyte surface.

### Example 4: Confirmation of apoptosis effect of 3T3-L1 cells due to single treatment of PLGA gas-generating nanoparticle (GNP) surface-modified by optimal peptides (P1 to P5)

In a 24-well plate, the 3T3-L1 cells were treated with the PLGA gas-generating nanoparticle surface-modified with the optimal peptide (P1 to P5) once at a concentration of 0.5 mg/ml, and then the medium was replaced and cultured for a total of 5 days. The 3T3-L1 cell decomposition effect was confirmed through Oil Red O staining, a staining method for staining the glycerol of 3T3-L1 cells. The 3T3-L1 cells were washed 3 times with PBS, fixed with 4 % formaldehyde for at least 1 hour, and then washed 3 times with PBS, then stained with filtered Oil Red O (0.3 %) solution for 20 minutes, and then washed with distilled water 3 times or more. Then, images were collected using a scanner (FIG. 3).

As shown in FIG. 3 , as a result of observing the remaining fat through Oil Red O staining, fat reduction was observed in the 3T3-L1 cells treated with the PLGA gas-generating nanoparticle surface-modified with the adipocyte-targeting or cell-penetrating peptides (P1 to P5), compared to the 3T3-L1 cells treated with the PLGA nanoparticle with non-modified surface or the PLGA gas-generating nanoparticle with non-modified surface.

### Example 5: Confirmation of apoptosis effect of 3T3-L1 cells due to multiple treatments of PLGA gas-generating nanoparticle (GNP) surface-modified with optimal peptides (P1, P3, P7)

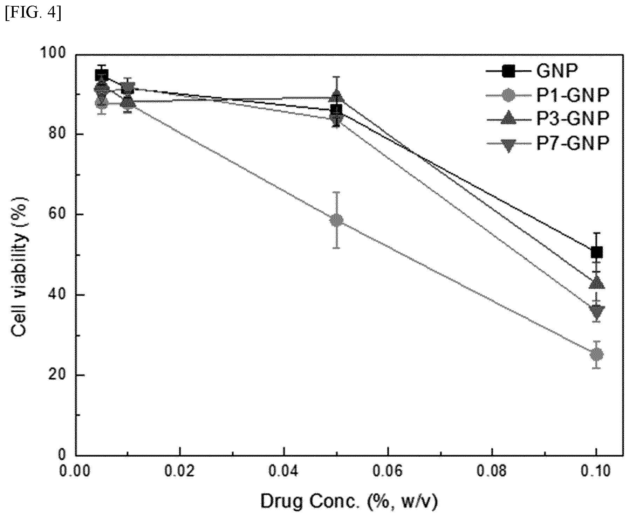

In a 96-well plate, the 3T3-L1 cells were treated with the PLGA gas-generating nanoparticle surface-modified with the optimal peptides [P1, P3, P7 (a molar ratio of a combination of P1 and P3 is 1:1)] at a drug (fine-grained calcium carbonate crystal) concentration of 0.005 to 0.1 (w/v) % twice in total for 8 days to confirm the apoptosis effect of the adipocytes due to the drug treatment. 20 µl of MTS(3-(4,5-dimethylthiazol-2-yl)-5-(3-carboxy-methoxyphenyl)-2-(4-sulfophenyl)-2H-tetrazolium, inner salt, MTS) was put into each well, and after incubation for 1 hour, absorbance was measured at 490 nm to check the apoptosis effect of the 3T3-L1 cells, and the result was expressed as a total percentage of viable cells compared to an untreated control (FIG. 4).

As shown in FIG. 4, it was confirmed that the 3T3-L1 apoptosis effect through two treatments of the PLGA gas-generating nanoparticle surface-modified with the adipocyte-targeting or cell-penetrating peptides [P1, P3, P7 (wherein a molar ratio of a combination of P1 and P3 1:1)] was superior to that of the PLGA gas-generating nanoparticle that are not surface-modified, In particular, in the case of the PLGA gas-generating nanoparticle surface-modified by the adipocyte-targeting peptide (P1), it was confirmed that the 3T3-L1 apoptosis effect through two treatments was significantly superior.

### Example 6: Confirmation of apoptosis effect of 3T3-L1 cells of PLGA gas-generating nanoparticle (GNP) surface-modified by optimal peptide (PI) of various molar ratios

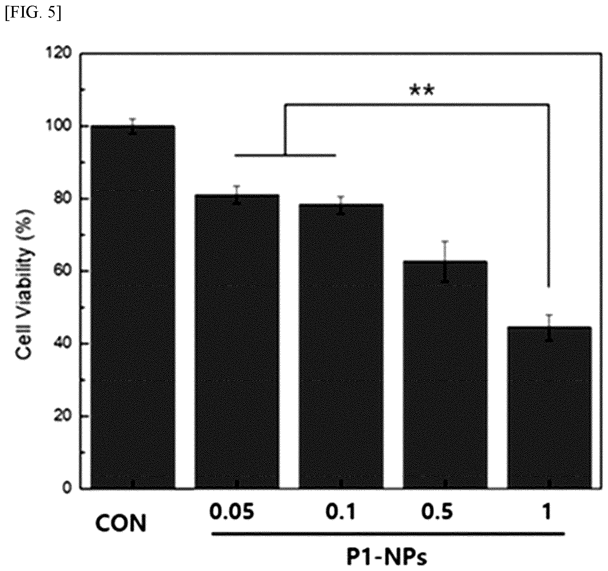

By varying the molar ratio of the optimal peptide (PI) (the molar ratio of the optimal peptide: PLGA = 0.05:1 to 1:1), the surface of the PLGA gas-generating nanoparticle (GNP) was modified, which was treated to the 3T3 cells with a drug (= fine-grained calcium carbonate crystal) concentration of 0.05 (w/v) % twice in total for 8 days in a 96-well plate to confirm the 3T3-L1 apoptosis effect due to the drug treatment (FIG. 5).

As shown in FIG. 5, it was confirmed that as the molar ratio of the adipocyte-targeting peptide (P1) increased, the 3T3-L1 apoptosis effect was increased, and the optimal molar ratio of the adipocyte-targeting peptide (P1) and PLGA was 0.5:1 to 1:1.

### Example 7: Confirmation of 3T3-L1 apoptosis effect according to various concentrations of PLGA gas-generating nanoparticle (GNP) surface-modified by optimal peptide (P1)

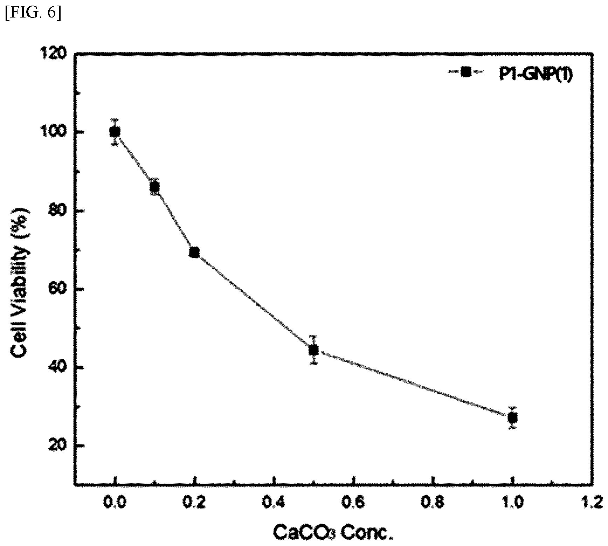

In a 96-well plate, the 3T3-L1 cells were treated with the PLGA gas-generating nanoparticle (GNP) surface-modified by the optimal peptide (P1) at various drug (=fine-grained calcium carbonate crystal) concentration of 0.0 to 1.0 (w/v) % twice in total for 8 days to confirm the 3T3-L1 apoptosis effect due to the drug treatment (FIG. 6).

As shown in FIG. 6, it was confirmed that as the concentration of the fine-grained calcium carbonate crystal increased, the 3T3-L1 apoptosis effect increased, and the optimal concentration thereof was 0.05 (w/v) % or more.

### Example 8: Confirmation of selective 3T3-L1 apoptosis effect of PLGA gas-generating nanoparticle (GNP) surface-modified by optimal peptide (P1)

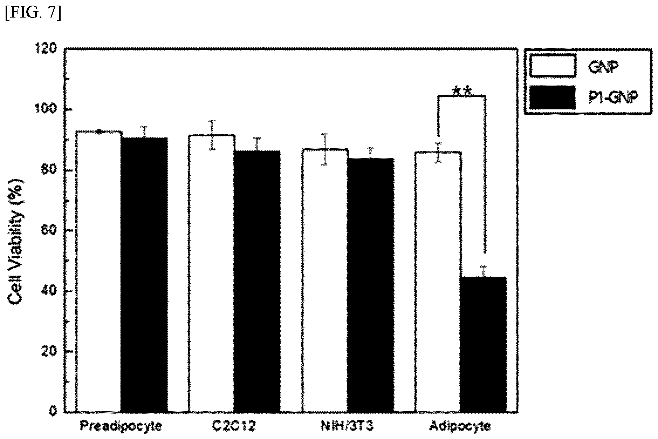

In a 96-well plate, the PLGA gas-generating nanoparticle (GNP) surface-modified with the optimal peptide (P1) was added to 3T3-L1 cells, 3T3-L1 progenitors, NIH3T3 fibroblasts and C2C12 myocytes at various drug (= fine-grained calcium carbonate crystal) concentration of 0.05 (w/v) % twice in total for 8 days to confirm the 3T3-L1 apoptosis effect due to the drug treatment (FIG. 7).

As shown in FIG. 7, it was confirmed that in the case of the PLGA gas-generating nanoparticle (GNP) surface-modified by the adipocyte-targeting peptide (P1), the 3T3-L1 apoptosis effect was excellent, but the apoptosis effect of 3T3-L1 progenitor cells, NIH3T3 fibroblasts and C2C12 myocytes was insignificant. That is, only the 3T3-L1 cells may be selectively killed.

### Example 9: Confirmation of fat reduction effect of PLGA gas-generating nanoparticle (GNP) surface-modified by optimal peptide (P1) in diet-induced obese mice

5-week-old C57BL/6 male mice were purchased from Orient Bio (Korea). Mice were bred with only 60 kcal % high-fat diet from 7 weeks after acclimatization period of 2 weeks and high-fat diet adaptation period of 4 weeks. Diet-induced obese mice were bred in a climate-controlled environment on a 12-hour, light-dark cycle, and were allowed to freely consume food and water.

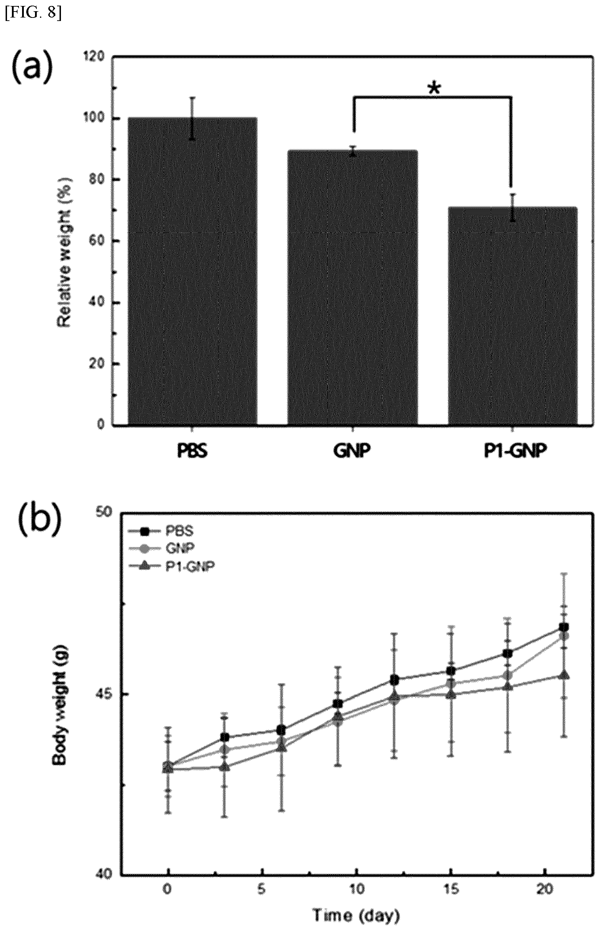

Specifically, in the right groin fat pad of diet-induced obese mice, 100 µl of the PLGA gas-generating nanoparticle (GNP) and the PLGA gas-generating nanoparticle (GNP) surface-modified by the optimal peptide (P1) (1 (w/v)%) were injected 5 times for 2 weeks. As a positive control, an equal volume of PBS was used. One week after the last injection, the diet-induced obese mice were sacrificed, and the weight was measured by excising the fat pad on the right side where the drug was injected and the left side without damage. The relative fat pad reduction in the diet-induced obese mice was obtained by the weight ratio of the fat pad on the right side injected with the drug to the fat pad on the left undamaged (FIG. 8).

As shown in FIG. 8, in the case of the group administered with the PLGA gas-generating nanoparticle (GNP) surface-modified by the adipocyte targeting peptide (P1), it was confirmed that the group showed a significant fat reduction effect without reducing the body weight of the subject.

The description of the present invention described above is for illustration, and those of ordinary skill in the art to which the present invention pertains will understand that it can be easily modified into other specific forms without changing the technical spirit or essential features of the present invention. Therefore, it should be understood that the embodiments described above are illustrative in all respects and not restrictive.

## Claims

1. A pharmaceutical composition for lipolysis, comprising:
a biocompatible polymer surface-modified by an adipocyte-targeting or cell-penetrating peptide; and
a surface-modified gas-generating nanoparticle containing fine-grained calcium carbonate crystal enclosed in the biocompatible polymer.

2. The pharmaceutical composition for lipolysis according to claim 1, wherein the surface-modified gas-generating nanoparticle is selectively introduced into adipocyte and then causes apoptosis of the adipocyte.

3. The pharmaceutical composition for lipolysis according to claim 1, wherein the surface-modified gas-generating nanoparticle does not cause apoptosis of cell other than the adipocyte.

4. The pharmaceutical composition for lipolysis according to claim 1, wherein the peptide consists of an amino acid sequence set forth in any one of SEQ ID NOs: 1 to 6.

5. The pharmaceutical composition for lipolysis according to claim 1, wherein the surface modification is formed by carbodiimide bonding between an amine group at an end of the biocompatible polymer and an amine group at an end of the peptide.

6. The pharmaceutical composition for lipolysis according to claim 1, wherein the biocompatible polymer is a polymer having a structure selected from the group consisting of polylactide (PLA), polyglycolide (PGA), polylactide-polyglycolide copolymer (PLGA), starch, glycogen, chitin, peptidoglycan, lignosulfonate, tannic acid, lignin, pectin, polyethylene glycol, polyethylene oxide, polyvinyl alcohol, polyethylene oxide-polypropylene oxide block copolymer, cellulose, hemicellulose, heparin, hyaluronic acid, dextran and alginate.

7. The pharmaceutical composition for lipolysis according to claim 1, wherein a molar ratio of the peptide and the biocompatible polymer is 0.05:1 to 1:1.

8. The pharmaceutical composition for lipolysis according to claim 1, wherein a concentration of the fine-grained calcium carbonate crystal in the pharmaceutical composition is 0.01 (w/v) % to 10 (w/v) %.

9. The pharmaceutical composition for lipolysis according to claim 1, wherein the pharmaceutical composition is in a form of an injection or transdermal administration.

10. A cosmetic composition for lipolysis, comprising:
a biocompatible polymer surface-modified by an adipocyte-targeting or cell-penetrating peptide; and
a surface-modified gas-generating nanoparticle containing fine-grained calcium carbonate crystal enclosed in the biocompatible polymer.
